Europäisches Patentamt

**European Patent Office**

Office européen des brevets

(11) Publication number : **0 366 211 B1**

# (12) EUROPEAN PATENT SPECIFICATION

(45) Date of publication of patent specification :
03.02.93 Bulletin 93/05

(51) Int. Cl.⁵ : **C07C 2/50,** C07C 5/25

(21) Application number : **89202696.4**

(22) Date of filing : **25.10.89**

(54) **Process for producing alpha olefins.**

(30) Priority : **27.10.88 US 263218
27.10.88 US 263225**

(43) Date of publication of application :
**02.05.90 Bulletin 90/18**

(45) Publication of the grant of the patent :
**03.02.93 Bulletin 93/05**

(84) Designated Contracting States :
**BE DE ES FR GB IT NL**

(56) References cited :
**US-A- 3 052 737
US-A- 3 534 116
US-A- 3 864 420**

(73) Proprietor : **SHELL INTERNATIONALE
RESEARCH MAATSCHAPPIJ B.V.
Carel van Bylandtlaan 30
NL-2596 HR Den Haag (NL)**

(72) Inventor : **Slaugh, Lynn Henry
12638 Rifleman Trail
Cypress Texas 77329 (US)**
Inventor : **Fong, Howard Lam-Ho
1707 Creekside Drive
Sugarland Texas 77478 (US)**

## Description

This invention relates to a process for producing an olefin product having an enhanced alpha olefin content from an olefin reactant comprising internal olefins or a mixture of internal and alpha olefins.

Many industrial processes produce olefins that are primarily internal olefins or are mixtures of alpha olefins and internal olefins. Due to the similarities in properties of alpha and internal olefins of the same molecular weight it is not an easy matter to separate the two. Olefins are frequently used as intermediates for the production of oil additives and detergents. The alpha and internal olefins can each be used to prepare end products having very different properties although the olefins utilized have the same molecular weight. Alpha olefins are particularly valued. A process that would enhance the alpha olefin content of an internal or a mixed alpha and internal olefin feedstock would be of considerable value.

U.S. patent No. 3,052,737, issued September 4, 1962, discloses reacting anthracene with vinylcyclohexene to produce an adduct which is then hydrogenated to convert the cyclohexene ring to a cyclohexane ring, followed by pyrolysis to produce vinylcyclohexane and anthracene. This reference does not suggest that anthracene would be useful in separating alpha and internal linear olefins.

The present invention provides a process for producing an olefin product having an enhanced alpha olefin content from an olefin feedstock comprising internal olefins or a mixture of internal and alpha olefins which process comprises:

(a) contacting said feedstock with an anthracene, and optionally a double-bond isomerization catalyst, at a temperature in the range of from 150 to 275°C to form an olefin adduct with the anthracene,

(b) separating said adduct from the product of step (a),

(c) heating said separated adduct at a temperature in the range of from 250 to 400°C to produce anthracene and an olefin product enhanced in alpha olefin content over the alpha olefin content of the feedstock, and

(d) separating anthracene from the product of step (c) to produce said product enhanced in alpha olefin.

Linear olefins are a preferred feedstock.

The feedstock olefins preferably used in the process of the instant invention are those olefins that are produced by commercial processes such as the oligomerization of ethylene, followed by isomerization and disproportionation. Some of the feedstocks may have had their alpha olefin content removed. These feedstocks are typically substantially linear olefins but may have smaller amounts of branched olefins present. The branched olefins may be separated out in a similar fashion as the linear olefins, the degree of separation being determined by the degree and location of the branching. Typically the feed olefins will have a carbon number ranging from 6 to 22, more preferably from 8 to 18. The physical properties of the olefins determine the suitable carbon numbers to be utilized. At the reaction temperature the olefins to be separated should be in the liquid or gaseous state rather than in the solid state. Olefins with carbon numbers greater than 18 and lower than 6 can be utilized in the instant process but from a commercially practical point of view feedstocks with carbon number ranging from about 6 to about 18 will be most frequently used.

An anthracene is used in the instant process to form the adduct primarily with the alpha olefin in the feedstock. As used herein "anthracene" refers to $C_{14}H_{10}$ (molecular weight 178.15) as well as substituted anthracenes possessing similar adducting properties as the unsubstituted anthracene, for example anthracenes bearing one, two or more simple substituents, such as lower alkyl, e.g., methyl, ethyl, butyl; halo, e.g., chloro, bromo, fluoro; nitro; sulfato; sulfonyloxy; carboxyl; carbo -lower-alkoxy, e.g., carbomethoxy, carbethoxy; amino; mono- and di-lower-alkylamino, e.g., methylamino, dimethylamino, methylethylamino; amido; hydroxy; cyano; lower-alkoxy, e. g., methoxy, ethoxy; lower-alkanoyloxy, e.g., acetoxy; monocyclic aryl, e.g., phenyl, xylyl, toluyl, benzyl. The particular substituents used should be inert under the reaction conditions and relatively small, such that they do not provide so much steric hindrance that the Diels-Alder reaction is inhibited. For example, 9-phenylanthracene is useful, whereas 9,10-phenylanthracene inhibits the Diels-Alder reaction. Suitable substituted anthracenes can be determined by routine experimentation.

The process of the instant invention is basically a three step process wherein (a) anthracene is reacted with the olefin, optionally in the presence of an olefin double-bond isomerization catalyst, to form an adduct primarily with alpha olefin, (b) the adduct is separated from the reaction mixture and (c) the adduct is pyrolized to release the alpha olefin enhanced product and regenerate the anthracene.

The (isomerization) Diels-Alder adduct forming reaction is carried out in a conventional fashion. It may be carried out continuously in a stirred tank reactor wherein olefin (and isomerization catalyst) and anthracene are added continuously to a stirred tank and a reaction product is continuously withdrawn from the stirred tank. Alternatively, the reaction may be carried out in a batch reactor, wherein the olefin (and isomerization catalyst) and the anthracene are charged to an autoclave which is then heated to reaction temperature to complete the reaction. The reaction is typically carried out at a temperature in the range of from 150 to 275°C, preferably from 200 to 250°C, and most preferably from 210 to 240°C. Pressures are not critical and typically are in the range of from atmospheric (1 bar) to 100 atmospheres (101 bar). The reaction can be carried out in the gas phase or liquid phase or mixed gas-liquid phase, depending on the volatility of the feed olefins.

Stoichiometric proportions or an excess of either olefin or anthracene can be used in forming the adducts but an excess of olefin is preferred. Advantageous ratios of about 1 to 2 moles of the olefin to the anthracene are preferred.

When an isomerization catalyst is used, it preferably has little or no polymerization or cracking activity. Suitable examples are phosphoric acid, both supported and unsupported, bauxite, alumina supported cobalt oxide, or iron oxide or manganese oxide, sodium and/or potassium on alumina, alkali metal promoted aluminas such as $K_2CO_3$ on alumina, supported platinum group metals, magnesium oxide, and calcium oxide. Other suitable isomerization catalyst are disclosed by the publication "Review of Olefin Isomerization" (H.N. Dunning, Industrial and Engineering Chemistry, 45, 551-564 (1953)).

An inert solvent can be used to dissolve the feed olefins or the anthracene or both in the reactor. Preferred solvents are the hydrocarbon solvents which are liquid at reaction temperatures and in which the olefins, anthracene and olefin-anthracene adducts are soluble. Illustrative examples of useful solvents include the alkanes such as pentane, iso-pentane, hexane, heptane, octane and nonane; cycloalkanes such as cyclopentane, and cyclohexane; and aromatics such as benzene, toluene, ethylbenzene and diethylbenzene. The amount of solvent to be employed can vary over a wide range without a deleterious effect on the reaction.

After the anthracene-olefin adduct has been formed, it is separated from the reaction mixture. The olefin-anthracene adduct is separated from the reaction mixture by conventional means. For example, it may be separated by flash distillation of the olefin to leave the adduct. Preferably, it is separated by cooling the reaction mixture until the adduct crystallizes out, followed by filtration or centrifugation to remove the unreacted olefin. In most cases the unreacted anthracene and isomerization catalyst will separate out with the adduct.

After the adduct has been separated from the reaction mixture there is left a residual product that still contains internal olefins and possibly a lesser amount of alpha olefins. This product may be recovered. Alternatively, it may be subjected to one or more additional reactions with a double-bond isomerization catalyst and anthracene or anthracene alone followed by separation of the adduct to further produce an alpha olefin enhanced product.

The final step of the instant process is to heat or pyrolyze the recovered olefin-anthracene adduct at a temperature in the range of from 250 to 400°C, preferably from 300 to 350°C. This pyrolysis frees the olefin from the anthracene. The anthracene is then separated from the resulting mixture to produce a second product enriched in alpha olefin content over that of the olefin feedstock. This separation is carried out by conventional means, e.g., flash distillation, filtration, centrifugation. This second reaction product may be subjected to one or more additional reactions with an isomerization catalyst and anthracene or with anthracene alone followed by separation of the adduct and pyrolysis of the olefin-anthracene adduct to produce a product having an even more enhanced alpha olefin content.

The present invention will now be illustrated by means of the following examples.

Examples 1 to 6

A 100 ml Parr autoclave was charged with 0.054 moles of anthracene (unsubstituted) and purged three times with argon and sealed. The autoclave was placed in a dry box and 0.212 moles of decene feed which had been nitrogen purged was added to the autoclave along with 20 ml of dry, nitrogen purged toluene. The autoclave was sealed, removed from the dry box, placed in a heating bath and heated to 220-230°C for 24 hours. The autoclave was stirred at 600 rpm during heating. The autoclave was the cooled to 20°C. The precipitated anthracene and decene-anthracene adduct was filtered out of the reaction product. The solid anthracene and decene-anthracene adduct were pyrolyzed in a nitrogen flow-pot heated to 300-350°C for 0.5 hours. After pyrolysis, the reaction product was filtered to separate the anthracene from a decene oil product. This product was analyzed by gas chromatography and ozonolysis. The results are shown in Table 1.

Additional experiments were carried out using various substituted anthracenes. The results of these experiments are shown in Table 1.

EP 0 366 211 B1

TABLE 1

| Example | Anthracene Used for Diels-Alder Adduct Formation | Temp. of Adduct Formation °C | RXN Time Hrs | Decene Composition of Feeds and Products of Adduct Pyrolysis | | | | |
|---|---|---|---|---|---|---|---|---|
| | | | | 1-Decene | 2-Decene | 3-Decene | 4-Decene | 5-Decene |
| | (N-decene feed) | - | - | 53.2 | 19.6 | 12.4 | 10.4 | 4.4 |
| 1 | Anthracene | 220-230 | 24 | 84.8 | 8.3 | 4.0 | 2.3 | 0.8 |
| 2 | 9,10-dichloro-anthracene | 220-230 | 24 | 77.0 | 14.8 | 4.7 | 2.7 | 0.8 |
| 3 | 9,10-dimethyl-anthracene | 220-230 | 24 | 93.8 | 5.1 | 0.7 | 0.3 | 0.1 |
| 4 | 9-methylanthracene | 220-230 | 24 | 88.1 | 7.7 | 2.5 | 1.2 | 0.5 |
| 5 | 9-phenylanthracene | 220-230 | 24 | 69.3 | 15.8 | 7.6 | 5.6 | 1.7 |
| 6 | 2-t-butylanthracene | 220-230 | 24 | 85.0 | 8.1 | 3.7 | 2.4 | 0.8 |

Example 7

The following example illustrates the use of an isomerization catalyst in the process of the instant invention. The isomerization catalyst used in the following example was a potassium carbonate/alumina catalyst prepared by adding 2.25 g of potassium carbonate dissolved in 5.8 ml of water to 12.75 g of Kaiser Alumina (KA-201), followed by drying and calcining in nitrogen at about 575°C for about 18 hours.

A 100 ml Parr autoclave was charged with 0.05 mol of 9,10-dimethylanthracene and purged three times with argon and sealed. The autoclave was placed in a dry box and 27.4 g (37 ml) of a decene mixture containing 1.7% 1-decene which had been nitrogen purged was added to the autoclave along with 20 ml of dry, nitrogen purged toluene and 2 g of the potassium/alumina catalyst described above. The autoclave was sealed, removed from the dry box, placed in a heating bath and heated to 240-260°C for 24 hours. The autoclave was stirred at 600 rpm during heating. The autoclave was then cooled to 20°C. The isomerization catalyst, precipitated anthracene and decene-anthracene adduct were filtered out of the reaction product. The filtrate was stripped by vacuum distillation to leave 11.5 g of residue. The filtrate was pyrolyzed in a nitrogen flow-pot heated to 335°C for 0.5 hours. After pyrolysis, the reaction product was filtered to separate the isomerization catalyst and anthracene from a decene oil product. This product was analyzed by gas chromatography and ozonolysis. The results are shown in Table 2.

### TABLE 2

#### Composition, Wt %

|  | 1-Decene | 2-Decene | 3-Decene | 4-Decene | 5-Decene |
|---|---|---|---|---|---|
| Feedstock | 1.7 | 40.7 | 25.8 | 21.3 | 10.5 |
| Product | 13.5 | 70.6 | 9.9 | 4.8 | 1.2 |

## Claims

1. A process for producing an olefin product having an enhanced alpha olefin content from an olefin feedstock comprising internal olefins or a mixture of internal and alpha olefins which process comprises:
   (a) contacting said feedstock with an anthracene, and optionally a double-bond isomerization catalyst, at a temperature in the range of from 150 to 275°C to form an olefin adduct with the anthracene,
   (b) separating said adduct from the product of step (a),
   (c) heating said separated adduct at a temperature in the range of from 250 to 400°C to produce anthracene and an olefin product enhanced in alpha olefin content over the alpha olefin content of the feedstock, and
   (d) separating anthracene from the product of step (c) to produce said product enriched in alpha olefin.

2. A process as claimed in claim 1, wherein step (a) is carried out at a temperature of from 200 to 250°C.

3. A process as claimed in claim 2 wherein step (a) is carried out at a temperature of from 210 to 240°C.

4. A process as claimed in any one of claims 1 to 3, wherein step (c) is carried out at a temperature of from 300 to 350°C.

5. A process as claimed in any one of claims 1 to 4, wherein the separations carried out in steps (b) and (d) are carried out by vacuum distillation.

6. A process as claimed in any one of claims 1 to 5, wherein the separations carried out in steps (b) and (d) are carried out by first cooling followed by filtration or centrifugation.

7. A process as claimed in any one of claims 1 to 6, wherein the feedstock olefins have carbon numbers in the range of from 6 to 22.

8. A process as claimed in claim 7, wherein the feedstock olefins have carbon numbers in the range of from 8 to 18.

9. A process as claimed in any one of claims 1 to 8, wherein the feedstock olefins comprise linear olefins.

10. A process as claimed in any one of claims 1 to 9, wherein a product comprising internal olefins is recovered from the residual product of step b).

**Patentansprüche**

1. Ein Verfahren zur Herstellung eines Olefinproduktes mit einem angereicherten Gehalt an Alpha-Olefin aus einem Olefin-Zuspeisungsmaterial, welches innere Olefine oder eine Mischung aus inneren und Alpha-Olefinen umfaßt, welches Verfahren die folgenden Stufen umfaát:
   a) Kontaktieren des Zuspeisungsmaterials mit einem Anthracen und gegebenenfalls einem Isomerisierungskatalysator für Doppelbindungen bei einer Temperatur im Bereich von 150 bis 275°C unter Bildung eines Adduktes aus dem Olefin und dem Anthracen,
   b) Abtrennen des Adduktes aus dem Produkt der Stufe (a),
   c) Erhitzen des abgetrennten Produktes auf eine Temperatur im Bereich von 250 bis 400°C zwecks Erzeugung von Anthracen und einem Olefin-Produkt, dessen Gehalt an Alpha-Olefin gegenüber dem Alpha-Olefin-Gehalt des Zuspeisungsmaterials erhöht ist, und
   d) Abtrennen von Anthracen aus dem Produkt der Stufe (c) zwecks Gewinnung von besagtem, an Alpha-Olefin angereichertem Produkt.

2. Ein Verfahren wie in Anspruch 1 beansprucht, in welchem die Stufe (a) bei einer Temperatur im Bereich von 200 bis 250°C durchgeführt wird.

3. Ein Verfahren wie in Anspruch 2 beansprucht, in welchem die Stufe (a) bei einer Temperatur im Bereich von 210 bis 240°C durchgeführt wird.

4. Ein Verfahren wie in irgendeinem der Ansprüche 1 bis 3 beansprucht, in welchem die Stufe (c) bei einer Temperatur im Bereich von 300 bis 350°C durchgeführt wird.

5. Ein Verfahren wie in irgendeinem der Ansprüche 1 bis 4 beansprucht, in welchem die Abtrennungsmaßnahmen in den Stufen (b) und (d) mittels Vakuumdestillation durchgeführt werden.

6. Ein Verfahren wie in irgendeinem der Ansprüche 1 bis 5 beansprucht, in welchem die Abtrennungsmaßnahmen in den Stufen (b) und (d) zuerst durch Kühlen und nachfolgend durch Filtrieren oder Zentrifugieren durchgeführt werden.

7. Ein Verfahren wie in irgendeinem der Ansprüche 1 bis 6 beansprucht, in welchem die zugespeisten Olefine Kohlenstoffzahlen im Bereich von 6 bis 22 aufweisen.

8. Ein Verfahren wie in Anspruch 7 beansprucht, in welchem die zugespeisten Olefine Kohlenstoffzahlen im Bereich von 8 bis 18 aufweisen.

9. Ein Verfahren wie in irgendeinem der Ansprüche 1 bis 8 beansprucht, in welchem die zugespeisten Olefine linear gebaute Olefine umfassen.

10. Ein Verfahren wie in irgendeinem der Ansprüche 1 bis 9 beansprucht, in welchem ein innere Olefine umfassendes Produkt aus dem Rückstandsprodukt von Stufe (b) gewonnen wird.

**Revendications**

1. Procédé pour la production d'un produit oléfinique ayant une teneur accrue en α-oléfine à partir d'une matière première oléfinique comprenant des oléfines internes ou un mélange d'oléfines internes et d'α-oléfines, lequel procédé comprend :
   (a) le contact de ladite matière première avec un anthracène et facultativement un catalyseur d'isomérisation de double liaison, à une température dans la gamme de 150 à 275°C, pour former un produit d'addition d'oléfine et de l'anthracène,
   (b) la séparation dudit produit d'addition du produit de l'étape (a),

(c) le chauffage dudit produit d'addition séparé à une température dans la gamme de 250 à 400°C pour produire l'anthracène et un produit oléfinique ayant une teneur accrue en α-oléfine par rapport à la teneur en α-oléfine de la matière première, et

(d) la séparation de l'anthracène du produit de l'étape (c) pour produire ledit produit enrichi en α-oléfine.

2. Procédé selon la revendication 1, dans lequel l'étape (a) est effectuée à une température de 200 à 250°C.

3. Procédé selon la revendication 2, dans lequel l'étape (a) est effectuée à une température de 210 à 240°C.

4. Procédé selon l'une quelconque des revendications 1 à 3, dans lequel l'étape (c) est effectuée à une température de 300 à 350°C.

5. Procédé selon l'une quelconque des revendications 1 à 4, dans lequel les séparations effectuées dans les étapes (b) et (d) sont effectuées par distillation sous vide.

6. Procédé selon l'une quelconque des revendications 1 à 5, dans lequel les séparations effectuées dans les étapes (b) et (d) sont effectuées tout d'abord par refroidissement, puis filtration ou centrifugation.

7. Procédé selon l'une quelconque des revendications 1 à 6, dans lequel les oléfines de la matière première ont des nombres d'atomes de carbone dans la gamme de 6 à 22.

8. Procédé selon la revendication 7, dans lequel les oléfines de la matière première ont des nombres d'atomes de carbone dans la gamme de 8 à 18.

9. Procédé selon l'une quelconque des revendications 1 à 8, dans lequel les oléfines de la matière première comprennent des oléfines linéaires.

10. Procédé selon l'une quelconque des revendications 1 à 9, dans lequel un produit comprenant des oléfines internes est récupéré à partir du produit résiduel de l'étape (b).